(19) Europäisches Patentamt
European Patent Office
Office européen des brevets

(11) Veröffentlichungsnummer: **0 428 956 A1**

(12) **EUROPÄISCHE PATENTANMELDUNG**

(21) Anmeldenummer: **90121529.3**

(22) Anmeldetag: **10.11.90**

(51) Int. Cl.5: **C08F 216/16**, C08F 222/16, C08F 222/06

(30) Priorität: **15.11.89 DE 3937982**

(43) Veröffentlichungstag der Anmeldung: **29.05.91 Patentblatt 91/22**

(84) Benannte Vertragsstaaten: **DE ES FR GB IT NL**

(71) Anmelder: **Giulini Chemie GmbH**
**Giulinistrasse 2 Postfach 150 480**
**W-6700 Ludwigshafen/Rhein(DE)**

(72) Erfinder: **Pelah, Zvi, Prof.**
**Savyion 56540**
**P.O. Box 3169(IL)**
Erfinder: **Potencsik, Istvan, Dr.**
**Am Steingarten 6**
**W-6800 Mannheim(DE)**
Erfinder: **Kopp, Werner, Dr.**
**Schönbrückstrasse 15**
**W-6721 Harthausen(DE)**
Erfinder: **Urmann, Ernst, Dr.**
**Limesstrasse 3**
**W-6700 Ludwigshafen(DE)**

(54) **Verfahren zur Herstellung von Copolymerisaten aus Maleinsäuremonoalkylestern und Vinylalkylethern.**

(57) Gegenstand der vorliegenden Erfindung ist ein neues Verfahren zur Herstellung von niederviskosen Copolymerisaten von Maleinsäuremonoalkylester und Vinylalkylethern, das dadurch gekennzeichnet ist, daß die Copolymerisation von Alkylvinylether mit einer Mischung, bestehend aus Maleinsäureanhydrid und Maleinsäuremonoalkylester durchgeführt wird. Die Steuerung der spezifischen Viskosität wird durch die Einstellung eines bestimmten Maleinsäureanhydrid/Maleinsäuremonoalkylester-Verhältnisses erreicht.

EP 0 428 956 A1

Abbildung 1

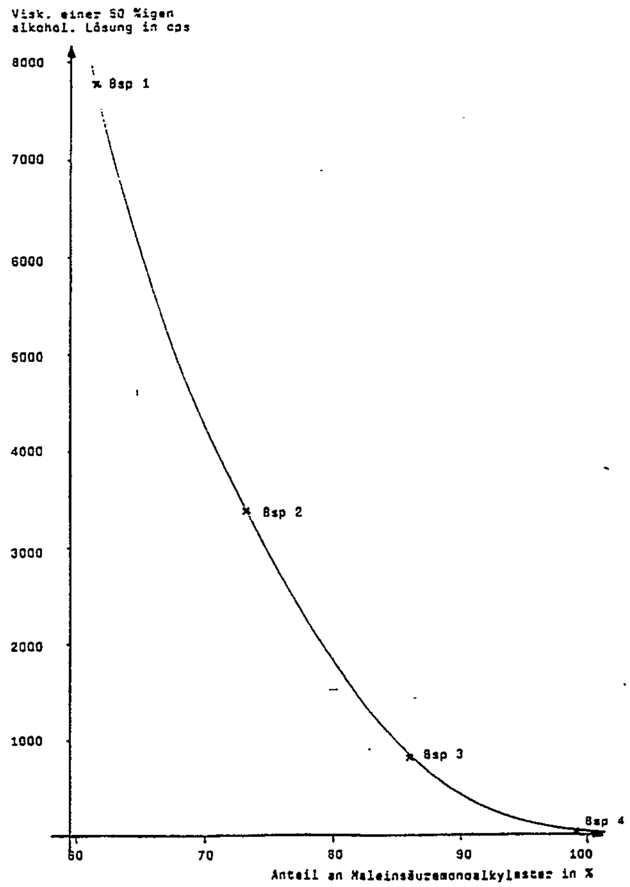
Visk. einer 50 %igen
alkohol. Lösung in cps
8000
7000
6000
5000
4000
3000
2000
1000
Bsp 1
Bsp 2
Bsp 3
Bsp 4
60
70
80
90
100
Anteil an Maleinsäuremonoalkylester in %

## VERFAHREN ZUR HERSTELLUNG VON COPOLYMERISATEN AUS MALEINSÄUREMONOALKYLESTERN UND VINYLALKYLETHERN

Gegenstand der vorliegenden Erfindung ist ein neues Verfahren zur Herstellung von niederviskosen Copolymerisaten von Maleinsäuremonoalkylester und Vinylalkylethern.

Copolymerisate von Maleinsäuremonoalkylestern und Vinylalkylethern sind im Stand der Technik bekannt. Sie finden Verwendung in der Kosmetik, vor allem in Haarsprays, worin sie als filmbildende Komponente enthalten sind. Bekannt sind in diesem Zusammenhang die Halbester mit Ethanol und Butanol, deren allg. Formel hier abgebildet ist, wobei $R^1 = CH^3$ und $R^2$ = Methyl oder Ethyl oder Propyl sein kann:

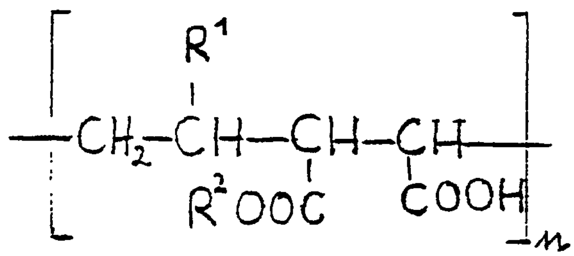

Zur Herstellung der Halbester sind in der Literatur bereits einige Verfahren beschrieben, wobei allen gemeinsam ist, das die Halbester durch Veresterung von Maleinsäureanhydrid/Alkylvinylether Copolymeratisaten gebildet werden, im folgenden MSA/AVE genannt. Zur Herstellung der MSA/AVE Copolymerisate gibt es ebenfalls zahlreiche Verfahren.

In einigen Verfahren wurden als Lösungsmittel Benzol oder Toluol eingesetzt, so z.B. in der US PS 27 82 182. Die Herstellung von hochmolekularen Maleinsäureanhydrid/Alkylvinylethern ist Gegenstand der GB PS 11 17 515. In diesem Verfahren wird Methylenchlorid als Lösungsmittel verwendet.

Es gibt in jüngerer Zeit auch Verfahren, in denen auf Lösungsmittel verzichtet werden konnte. So ist z. B. in der DE PS 37 12 265 ein Verfahren bekanntgemacht worden, das dadurch gekennzeichnet ist, das die radikalische Copolymerisation in einem Überschuß an Alkylvinylether durchgeführt wird, wobei hier der Alkylvinylether die doppelte Funktion als Lösungsmittel und als Reaktant übernimmt. Die Copolymerisate nach diesem Verfahren sind hochmolekular und eignen sich besonders zur Herstellung von Dentalhaftmitteln. Als Ausgangsmaterial zur Herstellung der Halbester eignen sie sich wegen ihrer hohen Viskosität jedoch nicht.

Verfahren zur Herstellung der Halbester wurden z.B. in den Patentschriften DE OS 19 30 009 und GB PS 12 33 468 bekanntgemacht. Da aber stets ein lösungsmittelhaltiges MSA/AVE Copolymere verestert wurde, enthalten die daraus gewonnenen Halbester zwangsläufig auch Lösungsmittel. Die Anwesenheit von selbst nur geringen Lösungsmittelresten ist, wie schon oben ausgefuhürt, in kosmetischen Formulierungen unerwünscht und in einigen Ländern sogar gesetzlich verboten.

Die Herstellung von Copolymerisaten von Maleinsäuremonoalkylestern und Alkylvinylethern ist Gegenstand der DE OS 37 33 158. Dieses Verfahren ist dadurch gekennzeichnet, daß in jeder Phase der Polymerisation die Vinylalkylether Komponente im Überschuß des Reaktionsgemisches vorliegt, die radikalische Copolymerisation in Aceton erfolgt und das Aceton während oder nach der Esterbildung bei Temperaturen bis 70 °C destillativ entfernt wird. Die nach diesem Verfahren hergestellten Produkte, die als al koholische Lösungen weiterverarbeitet werden, enthalten immer noch geringe Mengen Aceton (Spalte 5, Zeile 23), das in kosmetischen Formulierungen zu Hautreizungen führen kann, und darum unerwünscht ist.

Es bestand also weiterhin der Wunsch nach absolut lösungsmittelfreien und niedrigviskosen Halbestern, insbesondere filr kosmetische Formulierungen. Es wurde weiterhin nach einem Verfahren gesucht, nach dem sich die Viskosität der Halbester durch Variation der Reaktionsparameter einfach steuern läßt und die so hergestellten Produkte auch für den Anwendungsbereich Kosmetik geeignet sind. Die Aktualität dieses Gebiets unterstreichen auch die erst 1989 offengelegten Patentanmeldungen DE OS 37 33 158 und DE OS 37 36 996, wobei letztere ein Verfahren zur Herstellung von MSA/AVE Copolymerisaten betrifft.

Überraschenderweise konnten durch die vorliegende Erfindung die oben genannten Nachteile beseitigt werden. Sie ist dadurch gekennzeichnet, daß die Copolymerisation von Alkylvinylether mit einer Mischung, bestehend aus Maleinsäureanhydrid und Maleinsäuremonoalkylester durchgeführt wird. Das Verhältnis von Maleinsäureanhydrid zu Maleinsäuremonoalkylester liegt dabei zwischen 1 : 99 und 99 : 1. Die Polymerisation wird als radikalische Fällungspolymerisation durchgeführt, wobei die bekannten Radikalstarter eingesetzt werden und als Lösungsmittel die Vinylalkyletherkomponente, die im Überschuß vorliegt, sowohl als Lösungsmittel als auch als Comonomeres dient. Die vollständige Umsetzung zum Halbester kann mit niederen aliphatischen Alkoholen z. B. mit Methanol oder Ethanol oder Propanol oder Butanol erfolgen.

In der praktischen Durchführung wird zunächst der Maleinsäuremonoethylester separat hergestellt. Anschließend wird eine Mischung, bestehend aus dem Maleinsäuremonoethylester und Maleinsäureanhydrid hergestellt und diese Mischung in einen geeignetem Autoklaven überführt und der Radikalstarter hinzugefügt. Der Autoklav wird geschlossen, evakuiert und der Alkylvinylether eingefüllt. Nachdem die Ruhreinrichtung in Gang gesetzt wurde, wird die Temperatur über den Doppelmantel des Autoklaven innerhalb von 2 Stunden angehoben, bis alles Maleinsäureanhydrid gelöst ist. Die Heizung wird nun so eingestellt, das die Temperatur im Reaktionsraum alle 15 Min. um 1° C steigt. Nach 6 Stunden ist die Umsetzung vollständig. Der überschüssige Alkylvinylether wird abgezogen. Als Endprodukt verbleibt in Abhängigkeit vom Maleinsäureanhydrid : Maleinsäuremonoalkylether-Verhältnis entweder ein pulverförmiger Feststoff oder eine hochviskose, leichtgelbe Flüssigkeit, die mit Ethanol versetzt und unter Rückflußkühlung erhitzt wird. Es bildet sich so der Ethylvinylhalbester des Maleinsäureanhydrid/Methylvinylether Copolymers.

Die Steuerung der Kettenlänge des Copolymerisats und der spezifischen Viskosität wird durch die Einstellung eines bestimmten Maleinsäureanhydrid/Maleinsäuremonoalkylester-Verhältnisses erreicht, wie aus der beigefügten graphischen Darstellung und den Beispielen hervorgeht.

Natürlich kann man nach diesem Verfahren alle anderen Halbester z. B. Monobutylester, Mono-i-propylester etc. herstellen. Dazu wird von einem Gemisch bestehend aus Maleinsäureanhydrid und dem Halbester aus Maleinsäureanhydrid und dem jeweiligen Alkohol ausgegangen und das Reaktionspro dukt flach beendeter Copolymerisation in dem entsprechenden Alkohol gelöst und mit diesem am Rückfluß gekocht. Bei der Wahl der Etherkomponente sollten bevorzugt die niederen Alkylvinylether, die 1 bis 4 Kohlenstoffatome enthalten, eingesetzt werden.

In dem folgendem Beispiel A wird die Herstellung von Maleinsäuremonoethylester erläutert. In den Beispielen 2 bis 4 werden die erfindungsgemäßen Copolymerisate hergestellt, wobei das Maleinsäureanhydrid/Maleinsäuremonoalkylester Verhältnis jeweils variiert wird.

Beispiel 1

In einem 21-Dreihalskolben mit Rückflußkühler, Thermometer und KPG-Rührer werden 1000 ml Ethanol vorgelegt und nach Zugabe von 250 g Maleinsäureanhydrid zum Sieden unter Rückfluß erhitzt.

Dieser Zustand wird nach vollständiger Auflösung des Maleinsäureanhydrids noch für 30 min. beibehalten und dann der Rückflußkühler gegen eine Destillationsbrücke vertauscht. Der Überschuß an Ethanol wird abdestilliert. Die Ausbeute an Maleinsäuremonoethylester beträgt 358 g (97,5 % d. Th.).

Beispiel 2

In einem BÜCHI-Autoklav mit druckfestem 11 Glasgefäß, Ankerrührer und beheizbarem Doppelmantel werden 70 g des in Beispiel 1 hergestellten Maleinsäureethylesters und 30 g festes, gepulvertes Maleinsäureanhydrid vorgelegt. Nach Zugabe von 100 mg Dilaurylperoxid wird der Autoklav ge schlossen und evakuiert. Unter Eigendruck bzw. leichtem $N_2$ Überdruck werden 300 ml Methylvinylether eingefüllt und der Autoklav geschlossen. Der Rührer wird eingeschaltet und die Temperatur über den Doppelmantel innerhalb von 2 h von 21° C auf 40° C angehoben, bis alles Maleinsäureanhydrid gelöst ist. Die Heizung wird nun so eingestellt, das die Temperatur im Reaktionsraum alle 15 min. um ca. 1° C ansteigt. Nach 6 h sind monomeres Anhydrid und Halbester vollständig umgesetzt.

Der überschüssige Methylvinylether wird abgezogen und es bleiben 148 g einer hochviskosen gelben Flüssigkeit zurück. Sie wird mit 174 g Ethanol versetzt und zum Sieden unter Rückfluß erhitzt. Es bildet sich der Ethylhalbester des MSA/MVE Copolymers in 50 %iger ethanolischer Lösung mit einer Viskosität von 7800 cps (Brookfield RV, Sp. 4; 10 U/min.)

Die Beispiele 1 bis 4 sind in der Tabelle 1 zusammengefasst. Auf der Abbildung 1 kann der Zusammenhang zwischen Viskosität einer 50 %igen alkoholischen Lösung und dem Anteil an Maleinsäuremonoalkylester entnommen werden. Man sieht unmittelbar, das mit steigendem Maleinsäurehalbester Konzentration die Viskosität abnimmt.

Tabelle 1

| Beispiel-Nr. | 1 | 2 | 3 | 4 |
|---|---|---|---|---|
| Maleinsäureanhydrid [g] | 30 | 20 | 6 | 0,5 |
| [mol] | 0,306 | 0,204 | 0,061 | 0,005 |
| Maleinsäure-ethyl-halbester [g] | 70 | 80 | 54 | 70 |
| [mol] | 0,486 | 0,555 | 0,375 | 0,486 |
| MSA + MS-Ethyl-halbester [mol] | 0,792 | 0,759 | 0,436 | 0,491 |
| Methylvinylether [ml] | 300 | 300 | 150 | 150 |
| [g] | 225 | 225 | 112,5 | 112,5 |
| [mol] | 3,87 | 3,87 | 1,94 | 1,94 |
| Dilauroylperoxid [mg] | 100 | 100 | 70 | 70 |
| [mmol] | 0,251 | 0,251 | 0,176 | 0,176 |
| Verb. von MSA + MS-EHE : Dilauroylperoxid | 3155 : 1 | 3024 : 1 | 2477 : 1 | 2790 : 1 |
| Verb. voin MSA-Ethylhalbester [mol%] | 38,6 : 61,4 | 26,9 : 73,1 | 14,0 : 86,0 | 1,0 : 99,0 |
| Reaktionstemperatur [° C] | 60 | 60 | 60 | 60 |
| Reaktionsdruck [bar] | 3,8 - 4,3 | 3,8 - 4,3 | 3,8 - 4,3 | 3,8 - 4,3 |
| Reationszeit [b] | 5 | 5 | 5 | 5 |
| Visk. einer 50 %igen alkohol. Lsg. [cps] | 7800 | 3400 | 800 | 35 |

**Ansprüche**

1. Verfahren zur Herstellung von niederviskosen Copolymerisaten von Maleinsäureanhydrid mit Vinylalkylethern durch eine radikalische Fällungspolymerisation in einem Überschuß der Vinylalkyletherkomponente, dadurch gekennzeichnet, das als Comonomer neben der Vinylalkylkomponente eine Mischung, bestehend aus Maleinsäureanhydrid und Maleinsäuremonoalkylester, eingesetzt wird und das Verhältnis von Maleinsäureanhydrid zu Maleinsäuremonoalkylester in der Mischung zwischen 1 : 99 und 99 : 1 liegt, und die beiden Comonomeren in einer radikalischen Fällungspolymerisation unter Zuhilfenahme der üblichen Radikalinitiatoren bei Temperaturen zwischen 20 und 40° C polymerisiert werden, das Copolymerisat nach beendeter Reaktion vom überschüssigen Vinylalkylether befreit wird und dieses anschliessend mit einem niederen aliphatischen Alkohol der allg. Formel R- OH, in der R = Methyl oder Ethyl oder Propyl oder Butyl ist, vollständig zum Halbester umgesetzt wird.

2. Verfahren nach Anspruch 1, dadurch gekennzeichnet, das als Vinylalkylether die niederen Ether der allg. Formel R-O-R′, in denen R = Vinyl und R′ = Methyl oder Ethyl oder Propyl oder Butyl ist, eingesetzt werden.

3. Verfahren nach den Ansprüchen 1 und 2, dadurch gekennzeichnet, das zur Veresterung die niederen Alkohole, die 1 bis 4 Kohlen stoffatome enthalten, verwendet werden.

4. Verfahren nach den Ansprüchen 1 bis 3, dadurch gekennzeichnet, daß als Ether der Methylvinylether und zur Veresterung Ethanol eingesetzt werden.

5. Verwendung der nach den Ansprüchen 1 bis 4 hergestellten Copolymerisate in der Kosmetik.

**Abbildung 1**

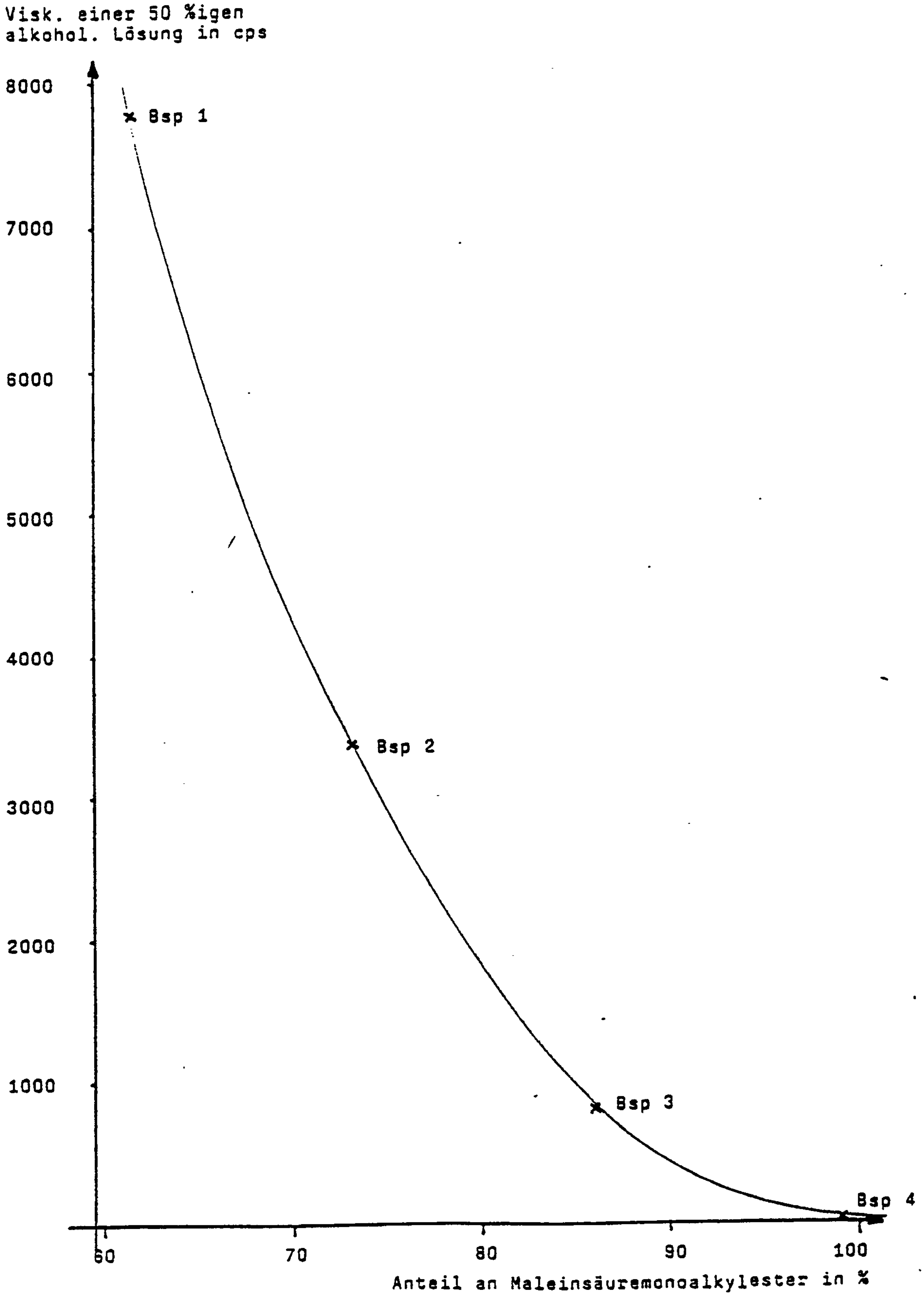
Visk. einer 50 %igen
alkohol. Lösung in cps
8000
7000
6000
5000
4000
3000
2000
1000
Bsp 1
Bsp 2
Bsp 3
Bsp 4
60
70
80
90
100
Anteil an Maleinsäuremonoalkylester in %

Europäisches Patentamt

# EUROPÄISCHER RECHERCHENBERICHT

Nummer der Anmeldung

EP 90 12 1529

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 289 713 (GIULINI CHEMIE GmbH)<br>* Insgesamt * | 1-5 | C 08 F 216/16<br>C 08 F 222/16<br>C 08 F 222/06 |
| Y,D | EP-A-0 310 079 (BASF AG)<br>* Insgesamt * | 1-5 | |
| A | GB-A- 701 992 (MONSANTO CHEMICAL CO.)<br>* Beispiel 1; Anspruch 1 * | 1-4 | |
| | | | **RECHERCHIERTE SACHGEBIETE (Int. Cl.5)** |
| | | | C 08 F |

Der vorliegende Recherchenbericht wurde für alle Patentansprüche erstellt

| Recherchenort | Abschlußdatum der Recherche | Prüfer |
|---|---|---|
| DEN HAAG | 06-02-1991 | GLIKMAN J-F.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)